(19) Europäisches Patentamt
European Patent Office
Office européen des brevets

(11) Publication number: **0 077 630**
**B1**

(12) # EUROPEAN PATENT SPECIFICATION

(45) Date of publication of patent specification: **17.04.85**

(51) Int. Cl.$^4$: **A 61 K 7/06**

(21) Application number: **82305413.5**

(22) Date of filing: **12.10.82**

(54) **Topical antimicrobial compositions.**

(30) Priority: **21.10.81 GB 8131752**

(43) Date of publication of application:
**27.04.83 Bulletin 83/17**

(45) Publication of the grant of the patent:
**17.04.85 Bulletin 85/16**

(84) Designated Contracting States:
**AT BE CH DE FR GB IT LI LU NL SE**

(56) References cited:
**GB-A- 807 787**
**US-A-3 940 482**

(73) Proprietor: **BEECHAM GROUP PLC**
**Beecham House Great West Road**
**Brentford Middlesex TW8 9BD (GB)**

(72) Inventor: **Dixon, Harold**
**164 Woodland Gardens**
**Isleworth Middlesex (GB)**
Inventor: **Day, Pamela Margaret**
**6 Raymead Way Fetcham**
**Leatherhead Surrey (GB)**

(74) Representative: **Cresswell, Thomas Anthony et al**
**European Patent Attorney Beecham Pharmaceuticals Great Burgh Yew Tree Bottom Road**
**Epsom Surrey KT18 5XQ (GB)**

**EP 0 077 630 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to topical antimicrobial compositions, such as shampoos, based on pyridine thione as antimicrobial agent.

Zinc pyridine thione (zinc OMADINE*; zinc 2-pyridine thiol-N-oxide, hereinafter referred to as ZPT) is well known as an antimicrobial agent and is frequently included in detergent compositions, especially shampoos for combatting the scalp microorganisms associated with dandruff. Japanese Patent Application No. 50—6171 (laid open no 51—82738 discloses the use of ethylene diamine tetra-acetic acid (EDTA) and alkali metal salts thereof, to improve the adsorption of ZPT onto the hair and scalp.

EDTA has the further effect of solubilising ZPT, thus ZPT/EDTA shampoos are clear whereas shampoos without EDTA consist of a suspension of ZPT particles and are opaque.

Other salts of pyridine thione are known and used as antimicrobial agents. Some of these are, like ZPT, insoluble, but may be solubilised using a strong chelating agent. Others are soluble, particular examples being sodium pyridine thione (NaPT) and pyridine thione disulphide.

It has now surprisingly been found that the antimicrobial effect of dissolved pyridine thione in topical antimicrobial formulations is enhanced by including a strong chelating agent and divalent copper cations in the formulation.

Accordingly the present invention provides a topical antimicrobial composition which comprises an aqueous or detergent solution of

(i) a pyridinethione salt or pyridine thione disulphide
(ii) a strong chelating agent and
(iii) from 0.007 to 1.5% w/v of divalent copper cations.

Pyridine thione disulphide has the formula:

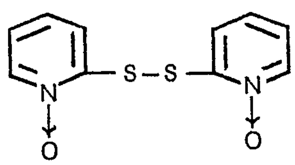

and is commercially available as an adduct with calcium chloride or magnesium sulphate (OMADINE CDS or OMADINE MDS respectively).

An aqueous solution of a pyridine thione salt may be obtained by dissolving a soluble pyridine thione salt, or may be obtained by solubilising an insoluble pyridine thione salt using a strong chelating agent. The chelating agent may be the same as that mentioned under item (ii) above, but it should be understood that a strong chelating agent is essential to the invention even when a soluble pyridine thione salt is employed.

Soluble pyridine thione salts are well known to the skilled person and include for instance sodium pyridine thione. Insoluble pyridine thione salts are also well known, a particular example being ZPT.

Strong chelating agents, suitable for use in the composition of the invention, are well known in the art and are usually di- or polyamines such as ethylene diamine tetra-acetic acid (EDTA), diethylene triamine penta-acetic acid (DTPA), tetraethylene triamine (TET), ethylene diamine (EDA) and diethylene triamine (DETA) or salts thereof. The preferred chelating agent is EDTA or the disodium salt thereof.

It is believed that solutions of pyridine thione undergo chemical changes, especially on exposure to sunlight or artificial light, and that these changes lead to the formation of the active species which is thought to be related to the pyridine thione disulphide. The antimicrobial properties of this material are potentiated by the combination of the strong chelating agent and divalent copper cations.

Compositions according to the present invention may be used to treat a variety of microbial conditions. Thus they may be presented as hair dressings, hair conditioners or hair shampoos for combatting scalp microorganisms such as those associated with dandruff, in particular *Pityrosporum ovale*. Other presentations of the composition include creams, ointments and lotions for treating scalp microorganisms, superficial mycoses and/or bacterial skin infections.

Suitably, compositions according to the present invention comprise from 0.005 to 5% w/w of pyridine thione excluding the weight of the cation component of the salt. Thus, when ZPT is used the compositions may comprise 0.01 to 2.0% w/w ZPT. When pyridine thione disulphide is used the compositions suitably comprise 0.01 to 2.0% w/w of pyridine thione disulphide excluding the weight of any salt combined in an adduct with the pyridine thione disulphide. Thus when, for instance, OMADINE MDS is employed, the compositions may comprise 0.01 to 2.0% w/w of OMADINE MDS.

Suitably the compositions comprise 0.01 to 5.0% w/w of a strong chelating agent. When an insoluble pyridine thione is employed, a strong chelating agent is also required in equimolar proportions, or in excess, to solubilise the salt. By way of example, from 0.01 to 5.0% w/w EDTA would be used in a composition comprising 0.01 to 2.0 w/w ZPT. If a soluble pyridine thione salt, or pyridine thione disulphide is used, the amount of strong chelating agent required may be reduced, since it is not necessary to

*OMADINE is a trade mark of the Olin Corporation.

solubilise the cation, nevertheless the amount of chelating agent would, suitably, still fall within the above range. The determination of optimum proportions of particular ingredients is, however, within the ability of the skilled person.

Conveniently the copper ions are provided by a simple inorganic copper salt such as copper nitrate or, preferably, copper sulphate. When copper sulphate is used, the above mentioned proportion of copper ions may be achieved using from 0.05 to 1.0% copper sulphate ($CuSO_4 \cdot 5H_2O$).

Accessory ingredients such as preservatives, colouring, perfume and conventional excipients in topical formulations may also be included in the compositions described above.

In a preferred embodiment of the present invention the compositions are presented as hair shampoos and comprise a detergent or mixture of detergents.

Accordingly the present invention, in a particular aspect, provides a shampoo composition comprising an aqueous or detergent solution of

(i) a pyridinethione salt or pyridine thione disulphide
(ii) a strong chelating agent and
(iii) from 0.007 to 1.5% w/w of divalent copper cations.

Suitably the shampoo comprises from 0.01 to 2% w/w of ZPT and from 0.01 to 5.0% w/w EDTA or an alkali metal salt thereof. Preferably the composition comprises from 0.05 to 1% w/w ZPT, most preferably about 0.2%, and from 0.05 to 5%, most preferably about 2.5% w/w of EDTA or an alkali metal salt thereof. Preferably the disodium salt of EDTA is used.

Suitably the composition comprises ZPT and copper ions in approximately equimolar proportions.

The most preferred compositions comprise about 0.2% w/w ZPT, about 2.5% w/w EDTA disodium salt and about 0.15% w/w copper sulphate.

The shampoo compositions described above may include any conventional detergent. Selection of such detergents is a matter of common general knowledge. For shampoo compositions it is usual to use an anionic detergent, for example alkali metal, ammonium or hydroxyalkylamine salts of alkyl sulphates, alkyl benzene sulphonates, alkyl sulphones, α-alkenylsulphonates, polyoxyethylenealkyl sulphonates and polyoxyethylenealkylphenyl sulphates. Suitable shampoo detergents therefore include sodium lauryl ether sulphate. Mixtures of detergents may also be used.

Shampoo compositions usually contain from 10 to 90% w/w of detergent or detergent mixture. More suitable the compositions contain from 10 to 16% w/w.

The shampoo compositions of the invention may also contain various conventional accessory ingredients including perfume, colouring, preservatives, foam builders, conditioning agents, materials to render the composition opaque, pearlescent or opalescent, and/or agents to adjust or maintain the pH of the composition.

Compositions of the invention may also be presented as hair dressings and lotions, ointments and creams. In each case the formulation comprises a pyridine thione salt or disulphide, a strong chelating agent and divalent copper cations dispersed in a conventional carrier. Typical carrier formulations are described in the literature—for instance in Harry's Cosmeticology 6th Edition, Leonard Hill Books, London (1973).

The compositions of the present invention may be produced by admixing the various constituents. When an insoluble pyridine thione salt is used as antimicrobial agent the salt and the strong chelating agent are admixed with the carrier and the mixture is allowed to clear and stand in the light for a period of days or weeks. Alternatively, the mixture may be exposed to artificial ultra violet light to hasten the process. The copper ions, in the form of a copper salt, are then admixed with the composition. When a soluble pyridine thione salt, or pyridine thione disulphide is used exposure to light is not necessary and the composition can be prepared simply by admixing the pyridine thione salt or pyridine thione disulphide with the carrier and adding divalent copper ions.

Various accessory ingredients may be added subsequently or at any convenient stage during this process.

It will be appreciated that, during the production of the composition, various chemical changes occur. Thus insoluble pyridine thione salts are solubilised by chelation of the metal cation and, it is believed, the pyridine thione moieties react together in light to form the antimicrobially active species.

Accordingly, the present invention provides a process for producing a composition as hereinbefore described which process comprises dissolving or solubilising a pyridine thione salt or pyridine thione disulphide and a strong chelating agent and admixing copper ions with the product.

When an insoluble pyridine thione salt is used the process suitably comprises the further step of exposing the mixture to ultra violet light prior to adding the copper ions.

The present invention will now be illustrated by the following Examples and Biological Data, which are not intended to limit the scope of invention in any way.

As used herein the term "SLES detergent solution" refers to a 27.5% w/v aqueous solution of sodium lauryl ether sulphate.

The following abbreviations have been employed in the Examples:—

| | |
|---|---|
| Zinc pyridine thione | ZPT |
| Pyridine thione disulphide magnesium sulphate adduct | MDS |
| Ethylene diamine tetraacetic acid | EDTA |

Proportions of ingredients are proportions by weight unless otherwise specified.
Compositions described in the Examples may be produced by conventional techniques.

Example 1

A detergent composition suitable for use as an antidandruff shampoo is formulated as follows:

| | % by weight |
|---|---|
| SLES detergent solution | 60 |
| Coconut diethanolamide | 4 |
| EDTA disodium salt | 2.5 |
| ZPT | 0.2 |
| Copper sulphate | 0.15 |
| U 834 Blue No 1 | 0.01 |
| Preservative | 0.01 |
| Distilled water | to 100 |

Sodium lauryl ether sulphate, coconut diethanolamide, water, EDTA disodium salt and ZPT are admixed and allowed to clear. The clear solution is left to stand in light at room temperature for 7 to 14 days, after which period the copper sulphate, colour and preservative are added.

The formulation remained as a clear blue liquid and retained full antimicrobial activity when stored at room temperature in sealed containers for 12 months.

Example 2

A water-in-oil antimicrobial composition may be prepared from the following ingredients:—

| | |
|---|---|
| ZPT or MDS | 0.02% |
| EDTA (Di-Sodium Salt) | 0.25% |
| Copper Sulphate | 0.015% |
| Mineral Oil | 18.0% |
| Beeswax | 3.0% |
| Ethoxylated Lanolin | 5.0% |
| Borax | 0.5% |
| Magnesium Sulphate | 0.1% |
| Perfume | qs |
| Water to | 100% |

Example 3

An oil-in-water composition may be prepared from the following ingredients:—

| | |
|---|---|
| ZPT or MDS | 0.02% |
| EDTA (Di-Sodium Salt) | 0.25% |
| Copper Sulphate | 0.015% |
| Stearic Acid | 3.0% |
| Propylene Glycol Mono Stearate | 1.0% |
| Mineral Oil | 4.0% |
| Glycerin | 2.5% |
| Sodium Carboxymethyl Cellulose | 0.3% |
| Triethanolamine | 1.0% |
| Perfume | qs |
| Water to | 100% |

Example 4

An antimicrobial composition may be prepared from the following ingredients:—

| | |
|---|---|
| ZPT or MDS | 0.02% |
| EDTA (Di-Sodium Salt) | 0.25% |
| Copper Sulphate | 0.015% |
| Stearic Acid | 3.0% |
| Propylene Glycol Mono Stearate | 1.0% |
| Polawax | 0.5% |
| Mineral Oil | 4.0% |
| Glycerin | 2.5% |
| Carbopol 934 | 0.5% |
| Triethanolamine | 1.0% |
| Perfume | qs |
| Water to | 100% |

Example 5

A mild astringent skin lotion may be prepared from the following ingredients:—

| | |
|---|---|
| ZPT or MDS | 0.02% |
| EDTA (Di-Sodium Salt) | 0.25% |
| Copper Sulphate | 0.015% |
| Glycerine | 5.0% |
| Rose Water | 15.0% |

| | |
|---|---|
| Alcohol (95%) | 30.0% |
| Methanol | 0.2% |
| Water to | 100% |

Example 6

An antidandruff hair conditioner may be prepared from the following ingredients:—

| | |
|---|---|
| ZPT or MDS | 0.02% |
| EDTA (Di-Sodium Salt) | 0.25% |
| Copper Sulphate | 0.015% |
| Stearyl Alcohol | 1.0% |
| Cetyl Alcohol | 2.0% |
| Glycerol Monostearate | 1.0% |
| Alcohol (Perfumery Quality) | 45.0% |
| Perfume | qs |
| Water to | 100% |

Biological data

*I.* The activity of various detergent and shampoo formulations, diluted 10 fold, against *Pityrosporum ovale* (NCTC 3074) was assayed using the disc diffusion method. *P. ovale* was grown on agar plates and incubated at 37°C. Zones of inhibition were measured after 48 hours. Results are given in Table I as an average of 5 replicates with the effect of a similar formulation containing only 1% w/w ZPT as a comparison.

i) Detergent formulation

| | % by weight |
|---|---|
| SLES detergent solution | 60 |
| ZPT | 0.2 |
| EDTA disodium salt | 2.5 |
| Copper sulphate ($CuSO_4 \cdot 5H_2O$, Analar) | See table 1 |
| Distilled water | to 100 |
| pH unadjusted, approx 5.0 | |

ii) Shampoo formulations

| | % by weight |
|---|---|
| SLES detergent solution | 60 |
| Coconut diethanolamide | 4.0 |
| ZPT | 0.2 |
| EDTA disodium salt | 2.5 |
| Copper sulphate ($CuSO_4 \cdot 5H_2O$, Analar) | See table 1 |
| 2-Bromo-2-nitropropane-1,3-diol (Bronopol) | 0.01 |
| U 834 Blue No 1 | 0.001 |
| Distilled water | to 100 |

pH unadjusted, approx 5.0

TABLE I
Mean zone of inhibition (mm)

| $CuSO_4 \cdot 5H_2O$ % by weight | Formulation Detergent | Shampoo |
|---|---|---|
| 0.0 | 5.08±0.54 | 5.7 ±1.5 |
| 0.05 | 9.13±0.88 | * |
| 0.1 | 10.75±0.58 | 11.14±0.65 |
| 0.15 | 10.2 ±0.67 | 12.24±0.72 |
| 0.2 | 10.27±1.05 | 12.58±0.76 |
| 0.25 | 11.38±0.56 | 11.68±0.8 |
| 0.3 | * | 11.52±0.56 |
| 1% ZPT | 6.74±0.42 | 6.77±0.76 |

*Not tested

*II.* The fungicidal activity of detergent formulations was assayed by contacting *P. ovale* (NCTC 3074) with the formulation for one hour followed by washing and membrane filtration. The colonies of *P. ovale* were then scored and counted. Results are given in Table II with control formulations for comparison.

Detergent formulation

| Formulation | % by weight 1 | 2 | 3 | 4 |
|---|---|---|---|---|
| SLES detergent solution | 60 | 60 | 60 | 60 |
| ZPT | 0.0 | 0.0 | 0.2 | 0.2 |
| EDTA disodium salt | 0.0 | 0.0 | 2.5 | 2.5 |
| Copper sulphate ($CuSO_4 \cdot 5H_2O$) | 0.0 | 0.3 | 0.0 | 0.3 |
| Distilled water | to 100 | 100 | 100 | 100 |

TABLE II
Fungicidal effect

| Formulation | $CuSO_4 \cdot 5H_2O$ (% by weight) | Score after one hour | % Kill |
|---|---|---|---|
| 1 | 0.0 | 6 | TNTC |
| 2 | 0.3 | 5 | TNTC |
| 3 | 0.0 | 5 | TNTC |
| 4 | 0.3 | 3½ | ca 50 |

TNTC: too numerous to count
Scoring:
3½ Colonies too numerous to count but well spaced
5 Heavy growth but not entirely confluent
6 Heavy confluent growth

*III. In vitro* Activity of various commercial antidandruff shampoo formulations was compared with the ZPT/EDTA/Copper combination evaluated in the *P. ovale* test system described above. Results are given in Table III.

TABLE III

| *Shampoo | Active ingredient | Zone of Inhibition (mm) |
|---|---|---|
| A | 0.2% ZPT/EDTA/Cu | 10.3±0.6 |
| B | 1% ZPT | 5.3±0.69 |
| C | 2.5 Selenium Disulphide | 7.4±0.42 |
| D | Coal Tar Extracts | <1 |
| E | Triclosan | <1 |

*Diluted 1:10 for testing

**Claims**

1. A topical antimicrobial composition which comprises an aqueous or detergent solution of
(i) a pyridine thione salt or pyridine thione disulphide
(ii) a strong chelating agent and
(iii) from 0.007 to 1.5% w/w of divalent copper cations

2. A composition as claimed in claim 1 comprising from 0.005 to 5% w/w of a pyridine thione salt excluding the weight of the cation component of the salt.

3. A composition as claimed in claim 1 or claim 2 comprising from 0.01 to 2.0% w/w of zinc pyridine thione.

4. A composition as claimed in claim 1 comprising from 0.01 to 2.0% w/w of pyridine thione disulphide.

5. A composition as claimed in any one of claims 1 to 4 comprising from 0.01 to 5.0% w/w of a strong chelating agent.

6. A composition as claimed in any one of claims 1 to 5 wherein the chelating agent is selected from ethylene diamine tetra-acetic acid, diethylene triamine penta-acetic acid, tetraethylene triamine, ethylene diamine, diethylene triamine and salts thereof.

7. A composition as claimed in any one of claims 1 to 6 comprising from 0.05 to 1.0% copper sulphate.

8. A composition as claimed in any one of claims 1 to 7 presented as a shampoo and comprising from 0.01 to 2% w/w zinc pyridine thione disulphide, from 0.01 to 5.0% w/w ethylene diamine tetra-acetic acid or an alkali metal salt thereof, from 0.05 to 1.0% w/w copper sulphate and from 10 to 90% w/w of detergent or detergent mixture together with conventional accessory ingredients.

9. A process for producing a composition as defined in claim 1 which process comprises dissolving or

solubilising a pyridine thione salt or pyridine thione disulphide and a strong chelating agent and admixing divalent copper cations with the product.

10. A process as claimed in claim 9 comprising the additional step of exposing the dissolved or solubilised pyridine thione salt or pyridine thione disulphide and strong chelating agent to ultra violet light prior to the addition of the copper ions.

**Patentansprüche**

1. Eine topische anti-mikrobielle Zusammensetzung, welche eine wäßrige Lösung oder eine Reinigungsmittellösung von einem
   (i) Pyridinthionsalz oder Pyridinthiondisulfid,
   (ii) einem starken Chelatbildner und
   (iii) von 0,007 bis 1,5 Gewichtsprozent zweiwertige Kupferkationen
enthält.

2. Eine Zusammensetzung wie in Anspruch 1 beansprucht, enthaltend von 0,005 bis 5 Gewichtsprozent eines Pyridinthionsalzes, ausschließlich des Gewichts der Kationenkomponente des Salzes.

3. Eine Zusammensetzung wie in Anspruch 1 oder 2 beansprucht, enthaltend von 0,01 bis 2,0 Gewichtsprozent Zinkpyridinthion.

4. Eine Zusammensetzung wie in Anspruch 1 beansprucht, enthaltend von 0,01 bis 2,0 Gewichtsprozent Pyridinthiondisulfid.

5. Eine Zusammensetzung wie in irgendeinem der Ansprüche 1 bis 4 beansprucht, enthaltend von 0,01 bis 5,0 Gewichtsprozent eines starken Chelatbildners.

6. Eine Zusammensetzung wie in irgendeinem der Ansprüche 1 bis 5 beansprucht, in welcher der Chelatbildner ausgewählt ist aus Äthylendiamin-tetraessigsäure, Diäthylentriamin-pentaessigsäure, Tetraäthylentriamin, Äthylendiamin, Diäthylentriamin und deren Salzen.

7. Eine Zusammensetzung wie in irgendeinem der Ansprüche 1 bis 6 beansprucht enthaltend von 0,05 bis 1,0% Kupfersulfat.

8. Eine Zusammensetzung wie in irgendeinem der Ansprüche 1 bis 7 beansprucht in Form eines Shampoos und enthaltend von 0,01 bis 2 Gewichtsprozent Zinkpyridinthiondisulfid, von 0,01 bis 5,0 Gewichtsprozent Äthylendiamintetraessigsäure oder ein Alkalimetallsalz davon, von 0,05 bis 1,0 Gewichtsprozent Kupfersulfat und von 10 bis 90 Gewichtsprozent eines Reinigungsmittels oder einer Reinigungsmittelmischung zusammen mit herkömmlichen Zusatzstofen.

9. Ein Verfahren zur Herstellung einer Zusammensetzung wie in Anspruch 1 definiert, welches Verfahren das Auflösen oder Löslichmachen eines Pyridinthionsalzes oder Pyridinthiondisulfids und eines starken Chelatbildners und das Zusetzen zweiwertiger Kupferkationen zu dem Produkt umfaßt.

10. Ein Verfahren wie in Anspruch 9 beansprucht, umfassend die zusätzliche Verfahrensstufe, daß das gelöste oder löslich gemachte Pyridinthionsalz oder Pyridinthiondisulfid und der starke Chelatbildner ultraviolettem Licht ausgesetzt werden, bevor die Kupferionen zugesetzt werden.

**Revendications**

1. Composition antimicrobienne locale, caractérisée en ce qu'elle comprend une solution aqueuse ou détergente
   (i) d'un sel de pyridine thione ou de disulfure de pyridine thione,
   (ii) d'un agent chélatant fort, et
   (iii) de 0,007 à 1,5% en poids/poids de cations de cuivre divalent.

2. Composition suivant la revendication 1, caractérisée en ce qu'elle comprend de 0,005 à 5% d'un sel de pyridine thione, à l'exclusion du poids du composant cationique du sel.

3. Composition suivant la revendication 1 ou la revendication 2, caractérisée en ce qu'elle comprend de 0,01 à 2,0% en poids/poids de pyridine thione de zinc.

4. Composition suivant la revendication 1, caractérisée en ce qu'elle comprend de 0,01 à 2,0% en poids/poids de disulfure de pyridine thione.

5. Composition suivant l'une quelconque des revendications 1 à 4, caractérisée en ce qu'elle comprend de 0,01 à 5,0% en poids/poids d'un agent chélatant fort.

6. Composition suivant l'une quelconque des revendications 1 à 5, caractérisée en ce que l'agent chélatant est choisi parmi l'acide éthylènediamine tétraacétique, l'acide diéthylène triamine penta-acétique, la tétraéthylène triamine, l'éthylène diamine, la diéthylène triamine et leurs sels.

7. Composition suivant l'une quelconque des revendications 1 à 6, caractérisée en ce qu'elle comprend de 0,05 à 1,0% en poids/poids de sulfate de cuivre.

8. Composition suivant l'une quelconque des revendications 1 à 7, présentée sous forme de shampooing, caractérisée en ce qu'elle comprend de 0,01 à 2% en poids/poids de disulfure de pyridine thione, de 0,01 à 5,0% en poids/poids d'acide éthylène diamine tétraacétique ou d'un sel de métal alcalin de celui-ci, de 0,05 à 1,0% en poids/poids de sulfate de cuivre et de 10 à 90% en poids/poids d'un détergent ou d'un mélange détergent, avec des constituants accessoires classiques.

9. Procédé de production d'une composition suivant la revendication 1, caractérisé en ce qu'il

comprend la dissolution ou la solubilisation d'un sel de pyridine thione ou du disulfure de pyridine thione et d'un agent chélatant fort, et le mélange de cations de cuivre divalent avec ce produit.

10. Procédé suivant la revendication 9, caractérisé en ce qu'il comprend une étape supplémentaire d'exposition du disulfure de pyridine thione ou du sel de pyridine thione dissous ou solubilisé et de l'agent chélatant fort, à une lumière ultraviolette, avant l'addition des ions cuivre.